# EUROPEAN PATENT SPECIFICATION

(11) **EP 1 267 995 B1**
(45) Date of publication and mention of the grant of the patent: **29.09.2004**
(21) Application number: 01918090.0
(22) Date of filing: 28.03.2001
(51) Int. Cl.: A61N 1/37

(54) **IMPLANTABLE HEART STIMULATOR**
IMPLANTIERBARER HERZSTIMULATOR
STIMULATEUR CARDIAQUE IMPLANTABLE

(30) Priority: 29.03.2000 SE 0001113
(43) Date of publication of application: 02.01.2003
(73) Proprietor: St. Jude Medical AB, 175 84 Järfälla (SE)
(72) Inventor: OBEL, Martin, S-182 33 Danderyd (SE); BUDGIFVARS, Göran, 163 51 Spanga (SE); UHRENIUS, Asa, 113 30 Stockholm (SE)
(86) International application number: PCT/SE2001/000680
(87) International publication number: WO 2001/072372

(56) References cited:
- US-A- 5 413 592
- US-A- 5 476 487
- US-A- 5 674 254
- US-A- 5 766 229

## Description

### Technical field of the invention

The present invention relates to an implantable heart stimulator.

### Background of the invention

Immediately after implantation of a heart stimulator and insertion of an heart electrode lead into the heart and attaching a stimulation electrode to heart tissue, the stimulation threshold, i.e. the least energy (pulse amplitude if the pulse width is constant) required to achieve heart contraction, is relatively high. After the first months following implantation of a heart stimulator the stimulation threshold eventually stabilizes at a more or less constant value in the order of some Volts (1-5 Volts).

Natural fluctuations of the stimulation threshold occur due to e.g. the activity of the patient (awake or asleep), the intake of drugs, etc.

A threshold search algorithm is implemented in many pacemakers of today that performs threshold searches either in response of one or many losses of capture or at timely intervals, e.g. 8 hours since last threshold search was performed.

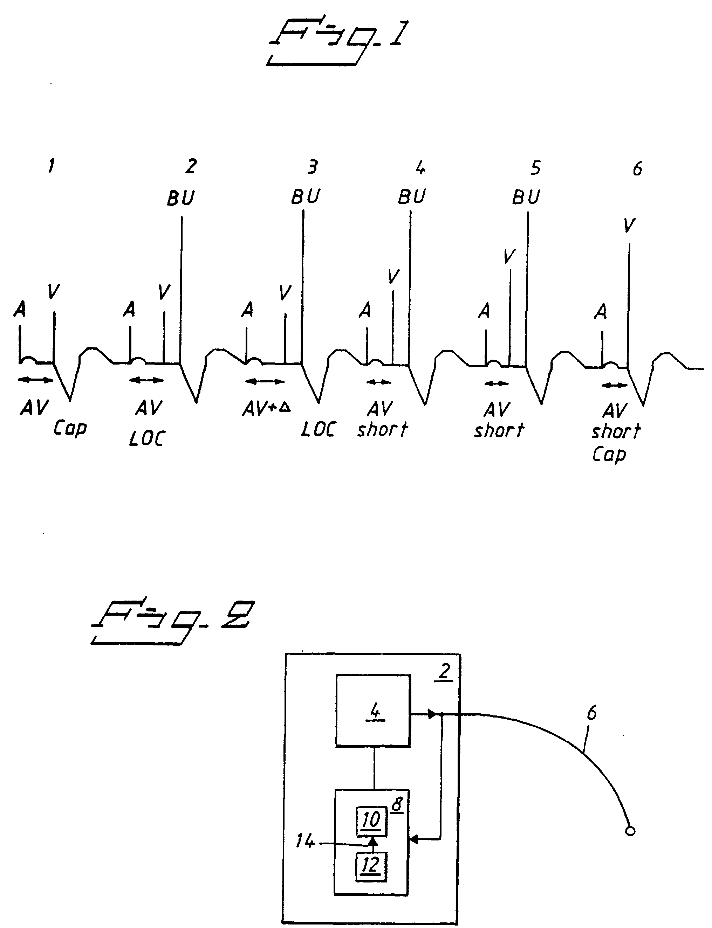

Figure 1 discloses an IEGM illustrating the principles of a threshold search algorithm according to established standard prior art, see e.g. US-5,476,487, and applicable in relation with the present invention. A and V designate atrial and ventricular stimulation pulses, respectively. BU is a high output backup pulse delivered if loss of capture (LOG) occurs. As can be seen (complex 3) the pre-programmed AV-interval is prolonged with Δ when a LOC occurs (complex 2). The reason for that is to await any intrinsic event if the first LOG was the result of a fusion beat. A fusion beat occurs when an intrinsic event more or less coincides with a generated stimulation pulse. In this case there is no intrinsic activity and the LOG was not a result of a fusion beat but was due to a changed stimulation threshold of the heart tissue, and a stimulation threshold search is initiated.
In figure 1 during the threshold search the pre-programmed AV-interval is shortened to "AV-short" to override any intrinsic heart activity. The ventricular stimulation amplitude is successively stepped up by a predetermined amplitude step of e.g. 0,1-0,3 V and each unsuccessful ventricular stimulation pulse is followed by a back-up pulse. This is performed until the stimulation threshold is detected, i.e. capture is detected from the ventricular stimulation pulse, and the stimulation pulse amplitude is then set to a value that equals the stimulation threshold plus a working margin, e.g. 0,3 V. As an alternative the ventricular stimulation amplitude may start at an amplitude above the stimulation threshold and then successively be stepped down until non-capture occurs.

It should be noted that the threshold search according to the established technique disclosed in the above-mentioned US-5,476,487 is performed by using a pre-programmed AVI shortened to "AVI-short" as indicated above.

US-5,766,229 is related to a capture verification method and apparatus for an implantable pacemaker utilizing heart rhythm stability measurements to minimized the likelihood of fusion. The heart rhythm stability is measured by determining a mean cardiac interval and a standard deviation of the measured intervals. If those values are acceptable (stable heart rhythm) the capture verification is performed.

In "User's manual for Microny SR+", pp 15-20, (Pacesetter AB, ordering no. 6324565 E500E, published in 1995) is an "Autocapture Pacing system" described where the threshold search is conducted every eight hour. A threshold search is also automatically conducted when two consecutive stimuli have resulted in no capture.
The algorithm is disabled when noise is detected and for as long as the noise is sensed.
Every threshold search requires the stimulation rate to be below the basic rate plus 20 min⁻¹.

As indicated above a stimulation threshold search might be performed either in response of loss of capture or at predetermined times.
The present invention is primarily directed to those threshold searches performed at a timely basis and not in response of detected loss of capture.

The object of the present invention is to achieve an improved implantable heart stimulator adapted to perform stimulation threshold searches that obtains a reliable result with a high degree of safety.

The invention is defined in claim 1.

Any embodiment which is in contradiction to the subject-matter of claim 1 is not part of the invention.

### Short description of the appended drawings

Figure 1 discloses an IEGM illustrating the principles of a threshold search algorithm according to established standard prior art.

Figure 2 discloses a schematic block diagram of an implantable heart stimulator 2 according to the invention.

### Detailed description of preferred embodiments of the invention.

Figure 2 discloses a schematic block diagram of an implantable heart stimulator 2 according to the invention. The heart stimulator comprises a pulse generating means 4 for generating stimulation pulses adapted to be applied to a heart via at least one electrode lead 6 and a control means 8. The control means comprises a threshold search means 10 for initiating a stimulation threshold search in order to determine a stimulation threshold of heart tissue and a threshold search timing means 12.
The threshold search timing means 12 generates a timing signal 14 at the same time every day to the threshold search means 10 in order to initiate threshold searches. According to a preferred embodiment of the invention is the timing signal generated every 8th hours, e.g. 00, 08, 16, 24 hours. The timing signal need not be generated at a regular interval but instead at predetermined times, e.g. at 05 and 23 o'clock.
The generation of the timing signal is not affected by threshold searches performed due to loss of capture.

When a timing signal is generated and applied to the threshold search means a stable condition index is determined that is related to the condition of the heart and is based on the result from one or many heart stability tests.

According to a preferred embodiment of the invention the heart stability test belongs to a first group of tests related to detection of parameters indicating emerging fusion beats.

A first heart stability test that belongs to the first group of tests fulfils its criterion for an acceptable result if a value of a detected evoked response signal following an applied stimulation pulse does not indicate a fusion beat. This value may represent e.g. the integral of the evoked response signal measured during an interval, e.g. 15-55 ms, following the stimulation pulse as for instance as disclosed in WO99/65569.

A second heart stability test that also belongs to the first group of tests fulfils its criterion for an acceptable result if an impedance signal representing the conditions inside the heart in combination with the timing of the stimulation pulse does not indicate a fusion beat.

According to another preferred embodiment of the invention a stability test belongs to a second group of tests related to the spontaneous activity of the heart. Almost all implantable heart stimulators of today are equipped with a sensing means adapted to sense evoked and spontaneous electrical activity of the heart. To perform the tests of the second group such sensing means arranged in the control means is utilised.

A third heart stability test that belongs to the second group fulfils its criterion for an acceptable result if the number of inhibited stimulation pulses is lower than a predetermined number during a time interval immediately before a threshold search is to be performed. If, for example, the predetermined number is set to 1 and the time interval is set to 1 minute the result of the test is not acceptable if one stimulation pulse is inhibited less than one minute before the threshold search is to be performed.

A fourth heart stability test that belongs to the second group of tests fulfils its criterion for an acceptable result if the stimulation rate during the threshold search is at least 10 bpm higher than the spontaneous rate immediately before the threshold search (V-V interval shorter than R-R interval). In a heart stimulator working according to the VVI-mode the spontaneous rate may be tested by temporarily slow down stimulation rate before the search shall be performed (increase V-V interval). If the spontaneous rate is only 0-10 bpm less than the intended stimulation rate during threshold search the intended stimulation rate is increased by 5-20 bpm (decrease V-V interval). The reason for stimulating with a higher rate is to override any spontaneous activity. In a heart stimulator working according to a DDD mode of operation the AV/PV delay is temporarily prolonged to seek spontaneous conduction. The conducted PR/AR interval must be at least 10-70 ms longer than the intended AV/PV intervals to be used during the threshold search.

A fifth heart stability test that also belongs to the second group of tests fulfils its criterion for an acceptable result if no PVC is detected.

A sixth heart stability test that also belongs to the second group of tests fulfils its criterion for an acceptable result if no spontaneous intervals is shorter than the intended threshold search interval during the e.g. 50 cardiac cycles preceding a threshold search. In a heart stimulator working according to the DDD/VDD-mode a tracked spontaneous rate must not exceed the base rate with more than a programmable value. The absolute rate shall not exceed 120 bpm.

A seventh heart stability test that not belongs to any of the above-mentioned groups fulfils its criterion for an acceptable result if a sensor indicated stimulation frequency (rate responsive frequency) not exceeds a basic stimulation frequency with more than a predetermined value, preferably 10 or 20 beats per minute.

The stable condition index is determined by the control means in dependence of the result(s) from at least one heart stability test. This may be performed in many different ways. According to a straightforward way of determining the index is a logical signal generated as a result of a heart stability test, whereas logical 1 indicates that the result of the stability test is acceptable and logical 0 indicates that the result not is acceptable. The logical signals are then applied at an AND-gate that generates a logical 1 as an output signal if all logical input signals are logical 1. According to an alternative way of determining the stable condition index is an analog value generated as a result of each test, the analog value, e.g. between 0-10, where values in the interval 7-10 is acceptable. All values from the different tests are then added together and an interval of the sum defining an acceptable overall result may be determined.

The invention is equally applicable for single chamber heart stimulators as for double chamber heart stimulators. The tests used is dependent of the presently used stimulation mode (e.g. VVI, VDD, DDI, DDD).

If the threshold search not is performed due to a non-acceptable value from any of the tests all the tests are repeated within a predetermined period of time, e.g. within 5 minutes. The time for generation of timing signals by the threshold search timing means is not altered in such cases.

The present invention is not limited to the above-described preferred embodiments. Various alternatives, modifications and equivalents may be used. Therefore, the above embodiments should not be taken as limiting the scope of the invention, which is defined by the appendant claims.

## Claims

1. Implantable heart stimulator (2) comprising a pulse generating means (4) for generating stimulation pulses adapted to be applied to a heart via at least one electrode lead (6), a threshold search timing means (12) that generates a timing signal at the same time(s) every day in order to initiate threshold searches at regular intervals, a control means (8) with threshold search means (10) for initiating a stimulation threshold search provided that a stable condition index fulfils predetermined criteria, wherein the stable condition index is related to the condition of the heart and is based on the result from at least one stability test, **characterized in that** said stability test is at least one of the following:
- A value of a detected evoked response signal following an applied stimulation pulse does not indicate a fusion beat.
- A value of a detected evoked response signal following an applied stimulation pulse does not indicate an emerging fusion beat.

2. Heart stimulator according to claim 1 **characterized in that** said stability test is fulfilled if the integrated evoked response signal does not indicate emerging fusion.

3. Heart stimulator according to claim 1 **characterized in that** said stability test is fulfilled if the integrated evoked response signal does not indicate fusion.

4. Heart stimulator according to claim 1 **characterized by** a stability test in which an impedance signal representing the conditions inside the heart in combination with the timing of the stimulation pulse does not indicate a fusion beat.

5. Heart stimulator according to claim 1, **characterized in that** said timing signal is generated at a regular interval, e.g. every 8th hours.

6. Heart stimulator according to claim 3, **characterized in that** the heart stability test fulfils its criterion for an acceptable result if no PVC is detected.

7. Heart stimulator according to claim 1, **characterized in that** a heart stability test fulfils its criterion for an acceptable result if a sensor indicated stimulation frequency not exceeds a basic stimulation frequency with more than a predetermined value, preferably 10-30 beats per minute.

## Patentansprüche

1. Implantierbarer Herzstimulator (2) mit einer Impulserzeugungsvorrichtung (4) zum Erzeugen von Stimulationsimpulsen, die geeignet sind einem Herzen über wenigstens eine Elektrodenleitung (6) zugeführt zu werden, mit einer Schwellensuch-Zeitsteuervorrichtung (12), die ein Zeitsteuersignal jeden Tag zu(r) gleichen Zeit(en) erzeugt, um Schwellenwertsuchvorgänge in regulären Intervallen einzuleiten, mit einer Steuervorrichtung (8), enthaltend eine Schwellenwertsuchvorrichtung (10) zum Einleiten einer Stimulationsschwellensuche, vorausgesetzt, dass ein stabiler Zustandsindex vorbestimmte Kriterien erfüllt, wobei der stabile Zustandsindex sich auf den Zustand des Herzens bezieht und auf dem Ergebnis aus wenigstens einem Stabilitätstest beruht, **dadurch gekennzeichnet, dass** der genannte Stabilitätstest wenigstens einer der folgenden ist:
- Ein Wert eines detektierten evozierten Reaktionssignals, das einem angewandten Stimulationsimpuls folgt, zeigt keinen Fusionsschlag an.
- Ein Wert eines detektierten Reaktionssignals, das einem angewandten Stimulationsimpuls folgt, zeigt keinen entstehenden Fusionsschlag.

2. Herzstimulator nach Anspruch 1, **dadurch gekennzeichnet, dass** der genannte Stabilitätstest erfüllt wird, falls das integrierte evozierte Reaktionssignal keine entstehende Fusion anzeigt.

3. Herzstimulator nach Anspruch 1, **dadurch gekennzeichnet, dass** der genannte Stabilitätstest erfüllt wird, falls das integrierte evozierte Reaktionssignal keine Fusion anzeigt.

4. Herzstimulator nach Anspruch 1, **gekennzeichnet durch** einen Stabilitätstest, bei dem ein Impedanzsignal, das den Zustand innerhalb des Herzens repräsentiert, in Verbindung mit der Zeitsteuerung des Stimulationsimpulses keinen Fusionsschlag anzeigt.

5. Herzstimulator nach Anspruch 1, **dadurch gekennzeichnet, dass** das Zeitsteuersignal in einem regelmäßigen Intervall, beispielsweise jede achte Stunde, erzeugt wird.

6. Herzstimulator nach Anspruch 3, **dadurch gekennzeichnet, dass** der Herzstabilitätstest sein Kriterium für ein akzeptierbares Ergebnis erfüllt, falls kein PVC detektiert wird.

7. Herzstimulator nach Anspruch 1, **dadurch gekennzeichnet, dass** ein Herzstabilitätstest sein Kriterium für ein akzeptierbares Ergebnis erfüllt, falls eine durch einen Sensor angezeigte Stimulationsfrequenz eine Grundstimulationsfrequenz nicht um mehr als einen vorbestimmten Wert, vorzugsweise 10-30 Schläge pro Minute, überschreitet.

## Revendications

1. Stimulateur (2) cardiaque implantable, comprenant des moyens (4) de production de stimulation d'impulsions aptes à être appliquées à un coeur par l'intermédiaire d'au moins une dérivation (6) à électrode, des moyens (12) synchronisés de recherche de seuil, qui produisent un signal de synchronisation au(x) même(s) moment(s) chaque jour, afin de faire débuter des recherches de seuil à des intervalles réguliers, des moyens (8) de contrôle ayant des moyens (10) de recherche de seuil pour faire débuter une recherche de seuil de stimulation, pourvu qu'un indice d'état stable satisfasse à des critères déterminés à l'avance, l'indice d'état stable étant en rapport avec l'état du coeur et étant fondé sur le résultat d'au moins un test de stabilité, **caractérisé en ce que** le test de stabilité est au moins l'un des suivants :
- une valeur de signal de réponse évoquée détecté suivant une impulsion de stimulation appliquée qui n'indique pas un complexe de fusion.
- une valeur d'un signal de réponse évoquée détecté suivant une impulsion de stimulation appliquée qui n'indique pas un complexe de fusion émergeante.

2. Stimulateur cardiaque suivant la revendication 1, **caractérisé en ce que** le test de stabilité est satisfait si le signal de réponse évoquée intégré n'indique pas de fusion émergeante.

3. Stimulateur cardiaque suivant la revendication 1, **caractérisé en ce que** le test de stabilité est satisfait si le signal de réponse évoquée intégré n'indique pas de fusion.

4. Stimulateur cardiaque suivant la revendication 1, **caractérisé par** un test de stabilité dans lequel un signal d'impédance représentant les conditions à l'intérieur du coeur, en combinaison avec le rythme de l'impulsion de stimulation, n'indique pas un complexe de fusion.

5. Stimulateur cardiaque suivant la revendication 1, **caractérisé en ce que** le signal de rythme est produit à un intervalle régulier, par exemple toutes les huit heures.

6. Stimulateur cardiaque suivant la revendication 3, **caractérisé en ce que** le test de stabilité du coeur satisfait son critère d'un résultat acceptable si un PVC n'est pas détecté.

7. Stimulateur cardiaque suivant la revendication 1, **caractérisé en ce qu'**un test de stabilité du coeur satisfait son critère d'un résultat acceptable si une fréquence de stimulation indiquée par un capteur ne dépasse pas une fréquence de stimulation de base de plus d'une valeur déterminée à l'avance, de préférence de 10 à 30 battements à la minute.
